# EUROPÄISCHE PATENTANMELDUNG

(11) **EP 1 146 066 A1**
(43) Veröffentlichungstag der Anmeldung: **17.10.2001**
(21) Anmeldenummer: 01103297.6
(22) Anmeldetag: 13.02.2001
(51) Int. Cl.: C08G 77/442, C08F 299/08, C08F 290/14, A61K 6/093

(54) **Ormocere, Verfahren zu deren Herstellung sowie Verwendung**

(30) Priorität: 31.03.2000 DE 10016324
(71) Anmelder: Degussa Dental GmbH & Co. KG, 63457 Hanau (DE)
(72) Erfinder: Loehden, Gerd, Dr., 63457 Hanau (DE); Albert, Philipp, Dr., 63456 Hanau (DE); Gall, Corina, 36381 Schlüchtern (DE); Borup, Björn, Dr., 80809 München (DE)

(57) **Zusammenfassung**

Die vorliegende Erfindung betrifft Ormocere erhältlich durch hydrolytische Kondensation einer oder mehrerer Siliciumverbindungen und anschließende Polymerisation organischer Monomere, wobei mindestens eine Siliciumverbindung Vinyletherreste der Formel (I) umfaßt 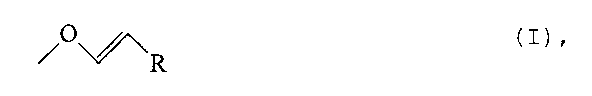 worin R Wasserstoff, Methyl oder Ethyl darstellen.

Die neuen Ormocere finden Anwendung in Dentalwerkstoffen.

## Beschreibung

Die vorliegende Erfindung betrifft Ormocere erhältlich durch hydrolytische Kondensation einer oder mehrerer Siliciumverbindungen, Verfahren zu deren Herstellung sowie Verwendung.

Ormocere sind eine neue Klasse von Verbundwerkstoffen, die aus atomaren Keramik- und Kunststoff-Netzen bestehen, welche sich miteinander verbinden und durchdringen. Der Name ORMOCER ist ein Kürzel für "ORganically MOdified CERamics".

Die Herstellung dieser an Silicone erinnernden Polymere erfolgt nach einem Sol-Gel-Prozess in Anwesenheit sauerer oder basischer Katalysatoren. Ormocere sind dementsprechend anorganisch-organische Polymere. Sie weisen im allgemeinen eine Oberfläche von 10 - 50 m²/g aus.

Ormocere sind an sich bekannt. So beschreibt beispielsweise WO 92/16 571 ein Verbundmaterial, das durch Hydrolyse von Siliciumverbindungen, die ethylenisch ungesättigten Gruppen enthalten, und anschließende Polymerisation der freigesetzten organischen Monomere gewonnen wird.

Von den organischen Monomeren ist beschrieben, daß diese entweder durch radikalische Initiatoren oder durch Ringöffnungspolymerisation ein organisches Netzwerk bilden. Offenbart sind insbesondere (Meth)acrylatverbindungen und Norbonenderivate.

Nachteilig bei Verwendung von (Meth)acrylaten ist insbesondere der Schrumpf, der durch die radikalische Polymerisation auftritt. Des weiteren sind freigesetzte Methacrylate gesundheitsschädlich.

Darüber hinaus wird die radikalische Polymerisation durch Luftsauerstoff inhibiert. Dies führt zur Bildung einer sog. Schmierschicht, die zu einer geringen Haftung der ausgehärteten Ormocere führen kann. Dieser Effekt ist insbesondere bei Verwendung der Ormocere als Füllmaterial in Dentalwerkstoffen unerwünscht.

Falls Monomere verwendet werden, die durch Ringöffnung polymerisiert werden, wird häufig eine geringe Reaktionsgeschwindigkeit erzielt. Darüber hinaus ist deren Umsatz relativ gering, so daß die mechanischen Eigenschaften des Verbundwerkstoffes nachteilig beeinflußt werden. Des weiteren können Färbungen je nach eingesetztem Ringöffnungskatalysator auftreten.

Darüber hinaus sind Ormocere bekannt, deren organische Monomere durch kationische Polymerisation von Trioxaspiroverbindungen erhalten werden. Diese Monomere zeigen gegenüber herkömmlichen Methacrylatmonomeren einen stark verringerten Schrumpf. Nachteilig hierbei ist jedoch, daß die Herstellung dieser Spiroverbindngen aufwendig ist und daher diese Monomere sehr teuer sind.

Diese Ormocere werden beispielsweise in DE-A-41 33 494 offenbart. Sie dienen als Dentalharzmassen, wie sie insbesondere zur Füllung von kariösen Kavitäten eingesetzt werden. Dementsprechend ist die geringe Polymerisationsgeschwindigkeit der Spiroetaverbindungen ein äußerst großer Nachteil.

Aufgabe der vorliegenden Erfindung war es deshalb, Ormocere anzugeben, deren organisches Netzwerk mit hoher Geschwindigkeit polymerisiert werden kann, ohne daß hierdurch eine hohe Volumenkontraktion auftritt.

Aufgabe der Erfindung ist weiterhin die Angabe von Verbundwerkstoffen der zuvor genannten Art, die nach Härtung der organischen Matrix eine hohe Abriebfestigkeit, hohe Härte, hohe Zähigkeit, hohe Druckfestigkeit und eine ausgezeichnete Kratzfestigkeit der Oberfläche aufweisen.

Darüber hinaus sollte das gehärtete Ormocer eine hervorragende Polierbarkeit zeigen.

Gegenstand der Erfindung ist des weiteren ein Verfahren zur Herstellung von Ormoceren sowie deren Verwendung.

Die Lösung dieser sowie weiterer im einzelnen nicht näher genannter Aufgaben, die sich jedoch für den Fachmann in naheliegender Weise aus dem Stand der Technik ergeben, gelingt durch ein Ormocer der eingangs erwähnten Art, welches die Merkmale des kennzeichnenden Teils des Anspruchs 1 aufweist. Zweckmäßige Ausgestaltungen des erfindungsgemäßen Ormocers werden in den von Anspruch 1 abhängigen Ansprüchen unter Schutz gestellt.

Hinsichtlich des Verfahrens und der Verwendung bieten die Ansprüche 9 und 10 einen Vorschlag für die Lösung der oben angegebenen Probleme an.

Dadurch, daß mindestens eine Siliciumverbindung Vinyletherreste der Formel (I) umfaßt 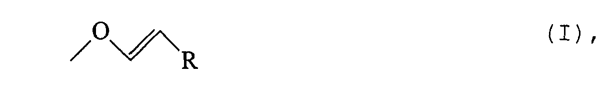 worin R Wasserstoff, Methyl oder Ethyl darstellen, gelingt es auf nicht vorhersehbare Weise Ormocere erhältlich durch hydrolytische Kondensation einer oder mehrerer Siliciumverbindungen und anschließende Polymerisation organischer Monomere zur Verfügung zu stellen, deren organisches Netzwerk mit hoher Geschwindigkeit härtbar ist, ohne daß hierdurch eine hohe Volumenkontraktion auftritt.

Darüber hinaus werden folgende Vorteile erzielt:
⇒ Die vollständig gehärteten Ormocere weisen eine hohe Haltbarkeit, eine hohe Druckfestigkeit, eine ausgezeichnete Polierbarkeit, eine hohe Kratzfestigkeit der Oberfläche, ein ausgezeichnetes Elastizitätsmodul sowie eine hohe Haftkraft beispielsweise an Zahnschmelz oder an Dentin auf.
⇒ Darüber hinaus sind die Ausgangsverbindungen erfindungsgemäßer Ormocere kostengünstig herzustellen und leicht erhältlich.
⇒ Des weiteren zeigen die Ormocere bei Härtung der organischen Matrix nur einen äußerst geringen oder keinen Schrumpf.
⇒ Ein weiterer Vorteil erfindungsgemäßer Ormocere besteht darin, daß die organische Matrix besonders leicht photochemisch gehärtet werden kann, wobei der Verbundwerkstoff vor Härtung der organischen Matrix besonders leicht zu bearbeiten ist.

Wie eingangs erwähnt, sind "Ormocere", die teilweise auch als "Ormosile" bezeichnet werden, Verbundwerkstoffe, die ein ineinander verflochtenes Netzwerk aus organischen und anorganischen Polymeren aufweisen. Der Ausdruck Netzwerk bezeichnet eine dreidimensionale Anordnung miteinander kovalent verbundener Stoffe. Hierbei füllt das organische Netzwerk Leerstellen des anorganischen Netzwerkes aus, so daß beide Netzwerke ineinander fest verbunden sind.

Anorganisch bedeutet in diesem Zusammenhang, daß die Hauptketten insbesondere aus -Si-O- Bindungen gebildet werden, die sowohl linear als auch verzweigt sein können. Die Si-Atome des anorganischen Netzwerkes können teilweise durch andere Metall- oder Halbmetallatome, wie beispielsweise Al, B, Zr, Y, Ba und/oder Ti ersetzt werden.

Das organische Netzwerk wird durch Polymerisation von organischen Monomeren, insbesondere von Vinyletherresten erhalten, wobei auch weitere Monomere, die mit Vinyletherresten der Formel (I) copolymerisierbar sind, umfaßt sein können.

Erhältlich ist das anorganische Netzwerk erfindungsgemäßer Ormocere durch hydrolytische Kondensation einer oder mehrerer Siliciumverbindungen, wobei bevorzugte Siliciumverbindungen Monomere Silane der Formel (II) 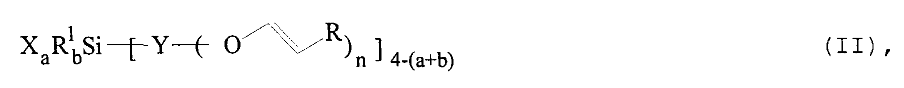 worin der Rest R unabhängig Wasserstoff, Methyl oder Ethyl, der Rest R¹ unabhängig ein aliphatischer, cycloaliphatischer oder aromatischer Rest mit 1 bis 30 Kohlenstoffatomen, der Rest X eine hydrolysierbare Gruppe und der Rest Y unabhängig einen unsubstituierte oder substituierte aliphatischen, cycloaliphatischen oder aromatischen Rest mit 1 bis 30 Kohlenstoffatome bedeuten, wobei eine oder mehrere CH₂-Gruppen durch O, C=O, -CO₂-,-SiR₂- und/ oder -SiR₂O- ersetzt sein können, und a eine ganze Zahl im Bereich von 1 bis 3, b eine ganze Zahl im Bereich von 0 bis 2 und n eine ganze Zahl im Bereich von 1 bis 3 darstellen, oder
cyclische, verzweigte oder lineare Oligo- bzw. Polysiloxane sind, umfassend Struktureinheiten der Formel (III) 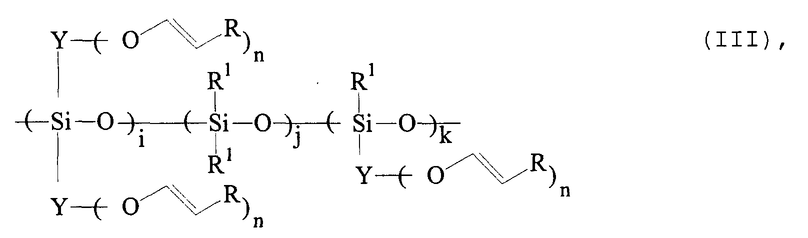 worin die Reste R, R¹ und Y sowie die Zahl n die zuvor genannte Bedeutung haben, i, j und k unabhängig eine ganze Zahl im Bereich von 0 bis 15 sind, wobei jedoch i und k nicht gleichzeitig 0 sein können.

Die aliphatischen Reste sind Alkenyl- und/oder Alkylreste mit 1-30, vorzugsweise mit 1-20 Kohlenstoffatomen und besonders bevorzugt mit 1-6 Kohlenstoffatomen, die gradkettig, verzweigt oder cyclisch sein können.

Spezielle Beispiele für Alkylreste sind Methyl, Ethyl, n-Propyl, i-Propyl, n-Butyl, s- Butyl, t-Butyl, i-Butyl, n-Pentyl, n-Hexyl.

Des weiteren werden Cycloalkylreste umfaßt, die ein oder mehrere Ringsysteme aufweisen. Spezielle Beispiele sind Cyclopentyl, Cyclohexyl und Norbonyl.

Zu den Alkenylresten gehören unter anderem Vinyl, Allyl, 2-Butenyl, Cyclopentenyl und Cyclohexenyl.

Darüber hinaus können die Silane der Formeln (I), (II) oder (III) auch aromatische Reste umfassen. Zu den bevorzugten Arylresten gehören Phenyl, Diphenylyl und Naphthyl.

Diese Reste können ggf. ein oder mehrere Substituenten tragen, z.B. Halogen, Alkyl, Hydroxyalkyl, Alkenyl, Alkoxy, Aryl, Aryloxy, Aralkyl, Acyloxy, Alkylcarbonyl, Alkoxycarbonyl, Furfuryl, Tetrahydrofurfuryl, Amino, Alkylamino, Dialkylamino, Trialkylamonium, Amido, Hydroxy, Formyl, Carboxy, Mercapto, Cyano, Nitro und Epoxyl.

Hydrolysierbare Gruppen sind Reste, die unter Einwirkung von Wasser freigesetzt werden. Hierzu gehören unter anderem Hydroxygruppen, Halogene, insbesondere Fluor, Chlor und Brom, Aryloxy-, Alkoxy- und/oder Acyloxy-Reste. Die hydrolysierbaren Gruppen werden in den Formeln (II) und (III) als X bezeichnet, wobei auch der Rest Y, je nach Aufbau unter Umständen durch Wasser freigesetzt werden kann.

Die Alkoxy-, Aryloxy-, Acyloxy- und Alkylcarbonylreste leiten sich vorzugsweise von zuvor genannten Alkyl- und Arylresten ab. Zu diesen gehören unter anderem Methoxy, Ethoxy, n- und i-Propoxy, n-, i-, s- und t-Butoxy, Acetyloxy, Propionyloxy, Methylcarbonyl, Ethylcarbonyl, Methoxycarbonyl, Benzyloxy, 2-Phenylethyloxy und Tolyloxy. Diese Reste können ebenfalls die zuvor genannten Substituenten aufweisen.

Spezielle Beispiele für Siliciumverbindungen der Formel (I) sind (4-(Vinyloxymethyl)cyclohexyl)methoxyethyl-trimethoxysilan; (4-(Vinyloxymethyl)phenyl)methoxyethyl-trimethoxysilan, 3,5-Divinyloxyphenyl-dimethyl-chlorsilan; 1,2-Di(4-(1-propenyloxymethyl)cyclohexyl)methoxyethyl-1,2-dimethyl-1,2-Dimethoxysiloxan und 1,3,5,7-Tetra-[(4-((1-propenyloxy)methyl)cyclohexyl)methoxyethyl]-1,3,5,7-tetramethylcyclotetrasiloxan. Diese Verbindungen können auch als Mischungen eingesetzt werden.

Die oben genannten Silane bzw. Siloxane sind Großteils kommerziell erhältlich, darüber hinaus können sie in an sich bekannter Weise synthetisch erhalten werden. Hierbei können die genannten Reste X und Y als Anhaltspunkte dienen. Hilfreiche Hinweise erfährt der Fachmann darüber hinaus beispielsweise aus Ullmann's Encyclopedia of Industrial Chemistry, 5. Aufl.

Die Herstellung von Vinylverbindungen aus Vinylhalogeniden und Alkoxiden wurde insbesondere von W. Reppe et al., *Justus Liebigs Ann*. *Chem.* **601** (1956) 81 - 110 beschrieben. Des weiteren sind Transvinylierungsreaktionen bekannt, bei denen eine Vinylgruppen eines Vinylethers oder Esters auf einen Alkohol bzw. eine Säure übertragen wird. Beispielhaft für viele seinen folgende Literaturstellen genannt: N. D. Field et al. : "Vinyl Ethers," in *High Polymers,* vol. 24, Wiley, New York 1971, pp. 365 - 411; S. A. Miller : "Vinyl Ethers," in *Acetylene*, *Its Properties*, *Manufacture and Uses,* vol. 2, Ernest Benn Ltd., London 1966, pp. 198 - 231; D. H. Lorenz : *Encyclopedia of Polymer Science and Technology,* vol. 14, Interscience, New York 1971, pp. 504 - 510.

Des weiteren können Vinylgruppen aus Allylgruppen durch Isomerisierungsreaktionen erhalten werden. Die Isomerisierung kann sowohl durch Addition von Base als auch durch Einwirkung von Ru-Katalysatoren, wie (Ph₃P)₃RuCl₂, erfolgen.

Die Darstellung Silicium-organischer-Verbindungen erfolgt häufig über Chlorsilane, die im allgemeinen kommerziell erhältlich sind. Diese können beispielsweise in etherischer Lösung mit Grignard-Reagenzien (z. B. RMgI) zu Alkyl- oder Arylsilanen umgesetzt werden. Zur Herstellung von Si-H-Bindungen enthaltenden Organosilanen kann die Reduktion der entsprechenden Organohalogensilane mit Lithiumaluminiumhydrid herangezogen werden, die in Hydrosilylierungsreaktionen Verwendung finden.

Die oben genannten Siloxane können beispielsweise aus den entsprechenden Hydrogensiloxanen durch Hydrosilylierung mit (1-Propenoxy)vinyloxyalkanen erhalten werden. Hierbei reagiert hauptsächlich die Vinyloxygruppe mit der Si-H-Bindung des Hydrogensiloxans. Zur Katalyse dieser Reaktion werden im allgemeinen Pt, Rh, und/ oder Pd-Verbindungen eingesetzt. Bekannt sind unter anderem Karsted und Wilkinson-Katalysatoren.

(1-Propenoxy)vinyloxyalkanen können beispielsweise durch Isomerisierung aus Allyloxyvinyloxyalkanen gewonnen werden. Wertvolle Hinweise hierzu findet der Fachmann beispielsweise in J.V.Crivello, G.Löhden: "Synthesis and Potopolymerisation of 1-Propenyl Ether Functional Siloxanes" in Chem. Mater., 1996, 8, 209-218.

Die Mischung, aus der die anorganischen Netzwerke erfindungsgemäßer Ormocere durch hydrolytische Kondensation erhältlich sind, können weitere organische Halbmetall- und Metallverbindungen aufweisen, die während der Hydrolyse in das anorganische Netzwerk eingebaut werden.

Zu diesen gehören unter anderem weitere Silane, die keine Vinylethergruppe der Formel (I) aufweisen.

Beispiele hierfür sind CH₃-Si-Cl₃, CH₃-Si-(OC₂H₅)₃, C₂H₅-Si-Cl₃, C₂H₅-Si-(OCH₃)₃, CH₂=CH-Si-(OC₂H₄OCH₃)₃, (CH₃)₂-Si-Cl₂, (CH₃)₂-Si-Br₂.

Weiterhin können die Mischungen, aus denen erfindungsgemäße Ormocere hergestellt werden können, hydrolysierbare Aluminiumverbindungen aufweisen, wobei bevorzugte Ausführungsformen durch die allgemeine Formel AlR²₃ dargestellt werden können, worin die Reste R² gleich oder verschieden Halogene, Alkoxy, Alkoxycarbonyl, Alkyl, Aryl und Hydroxy sein können. Besonders bevorzugte Reste ergeben sich aus den Gruppen, die beispielhaft für Siliciumverbindungen genannt wurden.

Zu bevorzugten Alumiumverbindungen der vorgenannten Art gehören Aluminiumalkoxyde und Aluminiumhalogenide. Spezielle Beispiele sind Al(OCH₃)₃, Al(OC₂H₅)₃, Al(OC₃H₇)₃, Al(OC₄H₉)₃, AlCl₃ und AlCl(OH)₂.

Des weiteren können hydrolysierbare Titan- oder Zirkoniumverbindungen mit den Siliciumverbindungen cohydrolisiet werden. Bevorzugte Verbindungen können durch die allgemeine Formel MXₒR¹ₜ dargestellt werden, worin M Ti oder Zr bedeutet, X und R¹ die in Formel (II) genannte Bedeutung haben, o eine ganze Zahl von 1 - 4 und t eine ganze Zahl von 0 - 3 darstellt.

Konkrete Beispiele für Zr- und Ti-Verbindungen sind TiCl₄, Ti(OC₂H₅)₄, Ti(OCH₃H₇)₄, Zr(OC₄H₉)₄, ZrCl₄, Zr(OC₂A₅)₄, Zr(OC₃H₇)₄, Zr(OC₄H₉)₄ und ZrOCl₂.

Darüber hinaus können auch Bor-, Zinn-, und Bariumverbindungen in die Ormocere eingebaut werden. Geeignete Verbindungen sind beispielsweise BCl₃, B(OCH₃)₃, B(OC₂H₅)₃, SnCl₄, Sn(OCH₃)₄, Ba(OCH₃)₃, Ba(OC₂H₅)₃ und Ba(OCOCH₃)₂.

Durch Einbau der schweren Elemente, insbesondere Zr, Ti oder Ba in die erfindungsgemäßen Ormocere kann die Röntgenopazität des Verbundwerkstoffs verändert werden. Des weiteren ändern sich hierdurch die mechanischen Eigenschaften des gehärteten Komposits.

Die mechanischen Eigenschaften lassen sich darüber hinaus durch das Verhältnis der leicht hydrolysierbaren Gruppen, die in den o.g. Formeln durch X dargestellt werden, zu den weniger leicht hydrolysierbaren Gruppen, die durch R¹ dargestellt sind, beeinflussen. Der Rest Y in den obigen Formeln kann je nach Aufbau hydrolysierbar oder nicht hydrolysierbar sein.

Je höher der Anteil der hydrolysierbaren Gruppen zu den nicht hydrolysierbaren Gruppen, desto härter aber auch spröder wird der Werkstoff. Vorzugsweise liegt das Verhältnis dieser Gruppen, das durch hydrolysierbar zu nicht hydrolysierbar gegeben ist im Bereich von 1:1 bis 3:1, vorzugsweise 1,5:1 bis 2:1, wobei diese Werte als Mittelwert über die als Mischung einsetzbaren hydrolysierbaren Verbindungen zu verstehen ist.

Die Herstellung der anorganischen Netzwerke kann in der auf dem Gebiet der Poly(hetero)kondensation üblichen Art und Weise erfolgen. Werden vorwiegend Siliciumverbindungen eingesetzt, genügt zur hydrolytischen Kondensation in den meisten Fällen ein Beimischen von Wasser, bei Raumtemperatur oder unter leichter Kühlung, wonach die resultierende Mischung einige Zeit gerührt wird.

Bei Anwesenheit von reaktiven Verbindungen, wie beispielsweise Metall-organischen Verbindungen von Aluminium, Titan oder Zirkonium, empfiehlt sich in der Regel eine stufenweise Zugabe des Wassers, wobei diese auch verdünnt erfolgen kann.

Als besonders geeignet hat sich in vielen Fällen die Eintragung der Wassermenge in das Reaktionsgemisch mit Hilfe von feuchtigkeitsbeladenen Adsorbentien, z.B. von Molekularsieben, und wasserhaltigen, organischen Lösungsmitteln, wie 80%iges Ethanol, erwiesen. Im allgemeinen wird die hydrolytische Kondensation bei Temperaturen zwischen -20 Grad Celsius und 130 Grad Celsius ,vorzugsweise zwischen 0 und 30 Grad Celsius durchgeführt. Die Umsetzung kann sowohl in Masse als auch in einem Lösungsmittel erfolgen. Geeignete Lösungsmittel sind u.a. aliphatische Alkohole, wie Ethanol oder i-Propanol, Ketone, insbesondere Dialkylketone, wie Aceton oder Methylisobutylketon, Ether, zu diesen gehören Diethylether oder Dibutylether, THF, und Ester, wie Essigsäureethylester.

Vinyletherreste der Formel (I) sind kationisch polymerisierbar. Dementsprechend findet die Herstellung der anorganischen Polymermatrix in neutralem oder basischem Medium statt. Dieses wird entweder durch ein basisches Lösungsmittel, wie beispielsweise Triethylamin, erzeugt oder durch Zugabe von basischen Hydrolyse- und Kondesationskatalysatoren, wie NH₃, NaOH, KOH und Methylimidazol.

Es hat sich insbesondere bei Verwendung von verschieden leicht hydrolysierbaren Verbindungen als bevorzugt erwiesen, nicht alle Ausgangsverbindungen zu Beginn der Hydrolyse vorzulegen, sondern nur einen Teil dieser Verbindungen mit Wasser zu kontaktieren und anschließend andere Verbindungen zuzugeben. Gleiches gilt für die Wasserzugabe, die in mehreren Stufen erfolgen kann, wobei nach jeder Wasserzugabe die Mischung für eine gewisse Zeit gerührt wird. Diese Vorgehensweise kann erforderlich sein, falls Teile der hydrolisierten Verbindungen zur Präzipitation neigen.

Die Kondensationzeit richtet sich nach jeweiligen Ausgangskomponenten und deren Mengenanteilen, dem ggf. verwendeten Katalysator, der Reaktionstemperatur usw. Im allgemeinen erfolgt die Polykondensation bei Normaldruck. Sie kann jedoch auch bei erhöhtem, oder bei verringertem Druck durchgeführt werden. Das so erhaltene Polykondensat kann entweder als solches oder nach Entfernung verwendeter oder gebildeter leicht flüchtiger Stoffe, wie beispielsweise Lösungsmittel, eingesetzt werden.

Beispielsweise kann das Polykondensat Dentalwerkstoffen zugegeben werden, bevor das organische Netzwerk durch geeignete Polymerisationskatalysatoren photochemisch und/oder thermisch gehärtet wird. Zum Entfernen von flüchtigen Bestandteilen kann die Reaktionsmischung bei vermindertem Druck und leicht erhöhter Temperatur, je nach Monomer bis ca. 80 Grad Celsius eingeengt werden.

Da die Polymerisation, wie nachfolgend beschrieben, vorzugsweise kationisch erfolgt, sollte nach einer basisch katalysierten Kondensation, der pH-Wert zumindest auf den Neutralpunkt gesenkt werden.

Vorzugsweise erfolgt die Bildung des organischen Netzwerks durch kationische Polymerisation. Dementsprechend werden den Polykondensaten oder den Mischungen, aus denen diese erhalten werden, Polymerisationskatalysatoren beigegeben, die vorzugsweise Lewis- oder Brönstett-Säuren bzw. Verbindungen, die solche Säuren freisetzen, wie beispielsweise BF₃ oder dessen etherische Addukte (BF₃·THF,BF₃·Et₂O, usw.), AlCl₃, FeCl₃, HPF₆, HAsF₆, HSbF₆, HBF₄, denen unter Umständen ein halogenierter Kohlenstoff, wie beispielsweise Triphenylchlormethan zugegeben wird, oder Substanzen, die nach Bestrahlen durch UV oder sichtbares Licht oder durch Wärme und/oder Druck die Polymerisation auslösen, wie z.B. (eta-6-Cumol)(eta-5-cyclopentadienyl)eisen-hexafluorophosphat, (eta-6-Cumol)(eta-5-cyclopentadienyl)eisen-tetrafluoroborat, substituierte Diaryliodoniumsalze und Triarylsulfoniumsalze.

Diesen Initiatoren werden üblicherweise Beschleuniger, wie beispielsweise Peroxyverbindungen, insbesondere vom Perestertyp, Benzoinderivate, Benzilverbindungen, oder Acylphosphinoxide beigefügt. Das Verhältnis von Initiator zu Beschleuniger kann in weiten Grenzen von 1 : 0,001 bis 1:10 variiert werden, vorzugsweise wird jedoch ein Verhältnis von 1:0,1 bis 3:6 verwendet.

Besonders bevorzugte Polymerisationskatalysatoren enthalten Iodoniumsalze als Initiatoren sowie Benzoinderivate, wie Benzoin, α-Methylbenzoinmethylether, α-Dicarbonylverbindungen, wie 2,3-Butandion, Campherchinon, Benzil und dessen Derivate, wie ω,ω-Dimethoxy-ω-phenylacetophenon, α-Hydroxyalkylphenonderivate, wie 1-Benzolycyclohexan-1-ol, sowie Acylphosphinoxidverbindungen, wie Benzoyldiphenylphosphinoxid, Trimethylbenzoyldiphenylphosphinoxid (weitere sind in EP 0 073 413 beschrieben) als Beschleuniger.

Besonders geeignete Diaryiodoniumverbindungen sind beispielsweise Diphenyliodoniumtetrafluoroborat, Diphenyiodoniumhexafluorophosphat, Bis-(4-methylphenyl) iodoniumhexafluorophosphat, Dinaphthyliodoniumhexafluoroantimonat, und Phenyl-4-methylphenyliodoniumhexafluoroantimonat.

Die Iodoniumsalze enthaltenden Polymerisationskatalysatoren lassen sich häufig durch UV-Licht im Wellenlängenbereich von 200 bis 400 Nanometer initiieren. Überraschend wurde festgestellt, daß diese Initiatoren Vinyletherreste der Formel (1) auch in den Polykondensaten besonders effektiv und schnell härten. Hierbei ist es nicht notwendig den Werkstoff während der gesamten Härtungszeit zu bestrahlen, da nach einer ausreichenden Initiierung der Dentalwerkstoff vollständig durchhärtet. Diese Eigenschaft ist besonders bei dem Einbringen von Füllungen nützlich.

Bevor das organische Netzwerk gebildet wird, können der hydrolysierbaren Siliciumverbindung bzw. dem hieraus hergestellten Polymerisat weitere ethylenisch ungesättigte, insbesondere kationisch polymerisierbare Monomere beigefügt werden.

Hierbei kann es sich sowohl um monofunktionale als auch um polyfunktionale Monomere handeln. Zu den monofunktionalen Monomeren gehören u.a. Vinylester, wie Vinylacetat, Vinylether, wie z.B. Propylvinylether, Butylvinylether, 2-Methylpropylvinylether, Pentylvinylether, Hexylvinylether, ((1-Propenoxy)ethyl)trimethylsilan, ((1-Propenoxy)ethyl)triethylsilan, und Vinylaromaten, wie z.B. Styrol, substituierte Styrole mit einem Alkylsubstituenten in der Seitenkette, wie z.B. α-Methylstyrol und α-Ethylstyrol, substituierte Styrole mit einem Alkylsubstituenten an dem Ring, wie beispielsweise Vinyltoluol und p-Methylstyrol, halogenierte Styrole, wie beispielsweise Monochlorstyrole, Dichlorstyrole, Tribromstyrole und Tetrabromstyrole.

Beispiele polyfunktionaler Monomere sind 1,3,5-Benzoltricarbonsäure-tris-4-(ethenyloxy)butanolester; Butandisäure-bis-(4-ethenyloxy)butanolester; Bis-[(4-((ethenyloxy)methyl)cyclohexyl)methyl]pentandioat; 2,2-Bis[4,1-phenyloxy4-((ethenyloxy)methyl)cyclohexyl)-methyl]propan; Diphenylether-4,4'-dicarbonsäure-(4-((ethenyloxy)methyl)cyclohexyl)methanoldiester und Diphenylether-4,4'-dicarbonsäure-2-(ethenyloxy)ethanoldiester.

Die erfindungsgemäßen Ormocere werden vorzugsweise dadurch hergestellt, daß man die Komponenten mischt, danach die Siliciumverbindungen basisch hydrolysiert und anschließend die ethylenisch ungesättigten Reste kationisch polymerisiert.

Wie zuvor erwähnt finden Ormocere hauptsächlich in Dentalwerkstoffen Anwendung. Dentalwerkstoff sind Materialien für die Zahnfüllung, Inlays oder Onlays, Zahnzemente, Glasionomerzemente, Kompomere, Verblendmaterialien für Kronen und Brücken, Materialien für künstliche Zähne, Dentinbondings, Unterfüllmaterialien, Wurzelfüllmaterialien oder sonstige Materialien für die prothetische, konservierende und präventive Zahnheilkunde. Insbesondere fallen unter den Begriff Dentalwerkstoff auch Komposits für zahnmedizinische und zahntechnische Einsatzzwecke, Versieglermaterialien, selbsthärtende Komposits, Stumpfaufbaumaterialien, Verblendkunststoffe, hoch- und normalgefüllte Dualzemente sowie normalgefüllte fluoridhaltige Zahnlacke.

Die erfindungsgemäßen Ormocere können ohne Zugabe weiterer Stoffe als Dentalwerkstoff eingesetzt werden. Zur Verbesserung der Verarbeitbarkeit, der mechanischen Eigenschaften und aus ästhetischen Gründen, können vor Polymerisation der ethylenisch ungesättigten Bindungen weitere Monomere, die oben genannt sind, als Bindemittel und weitere Füllstoffe zugegeben werden.

Die Verwendung anorganischer Füllstoffe in Dentalwerkstoffen ist an sich bekannt. Die Füllstoffe dienen insbesondere zur Verbesserung der mechanischen Eigenschaften. Als Füllstoff werden unter anderem Quarze, gemahlene Gläser, Aerosile, sphärische SiO₂-Partikel, die ggf. mit Titandioxid beschichtet sind, Zeolithe, schwer lösliche Fluoride, wie CaF₂, YF₃, Kieselgele sowie pyrogene Kieselsäuren oder deren Granulate weithin angewendet. Des weiteren können die erfindungsgemäßen Dentalwerkstoffe auch organische Füllstoffe, insbesondere Fasern aufweisen. Diese Füllstoffe sind im allgemeinen kommerziell erhältlich.

Insbesondere schwere Atome, wie Y und Zr, erhöhen die Röntgenopazität. Daher werden Füllstoffe bevorzugt, die diese Atome aufweisen.

Zum besseren Einbau in die Polymermatrix können diese Füllstoffe mit Haftverbesserungsmitteln behandelt werden. Hierzu sind unter anderem Silane, wie ((1-Propenoxy)ethyl)-trimethoxysilan, ((1-Propenoxy)ethyl)triethoxysilan oder (1-Propenoxy)ethyltrichlorsilan, geeignet.

Ohne daß hierdurch eine Beschränkung erfolgen soll weisen diese Füllstoffe im allgemeinen eine Korngröße im Bereich von 0,02 µm bis 100 µm, bevorzugt von 0,05 bis 10 µm und ganz besonders bevorzugt von 0,1 bis 5 µm auf, wobei die Form der Füllstoffe keiner besonderen Beschränkung unterliegt. Sie können dementsprechend beispielsweise kugelförmig, splitterförmig, plättchenförmig und/oder faserförmig sein.

Diese Füllstoffe können einzeln oder als Mischungen eingesetzt werden, wobei der Einsatz von Mischungen unter Umständen Verbesserungen hinsichtlich der Ästhetik und eine weitere Verbesserung der mechanischen Eigenschaften ermöglicht.

Der Füllstoffgehalt der Dentalwerkstoffe, einschließlich der erfindungsgemäßen Ormocere, liegt im Bereich von 1 bis 95 Gew.-%, bevorzugt im Bereich von 50 bis 90 Gew.-% und ganz besonders bevorzugt im Bereich von 65 bis 90 Gew.-%, bezogen auf das Gesamtgewicht. Ein hoher Füllstoffgehalt führt zu einem geringen Schrumpf, hervorragenden mechanischen Eigenschaften und zu einer guten Polierbarkeit des ausgehärteten Werkstoffs. Andererseits muß in dem Dentalwerkstoff genügend Bindemittel zur Härtung vorhanden sein. Je gleichmäßiger das Bindemittel in den Füllstoff eingearbeitet werden kann, desto höher kann der Füllstoffgehalt gewählt werden.

Darüber hinaus können die Dentalwerkstoffe der vorliegenden Erfindung Hilfsstoffe aufweisen. Hierzu gehören unter anderem Stabilisatoren, Pigmente oder Verdünnungsmittel.

Der ausgehärtete Dentalwerkstoff zeigt hervorragende Eigenschaften hinsichtlich der Biegefestigkeit, des Elastizitätsmoduls, der Druckfestigkeit, der Haltbarkeit und der Abriebfestigkeit.

Darüber hinaus zeigen die Dentalwerkstoffe der vorliegenden Erfindung eine ausgezeichnete Polierbarkeit, eine geringe Wasseraufnahme und hervorragende ästhetische Eigenschaften, insbesondere hinsichtlich der Transparenz und des Brechungsindexes.

Die nachfolgenden Beispiele und Vergleichsbeispiele dienen zur genaueren Erläuterung der Erfindung, ohne daß diese hierauf beschränkt werden soll. Die nachfolgenden Prozentangaben beziehen sich auf das Gesamtgewicht, es sei denn anderes ist vermerkt.

## Patentansprüche

1. Ormocere erhältlich durch hydrolytische Kondensation einer oder mehrerer Siliciumverbindungen und anschließende Polymerisation organischer Monomere, **dadurch gekennzeichnet, daß** mindestens eine Siliciumverbindung Vinyletherreste der Formel (I) umfaßt 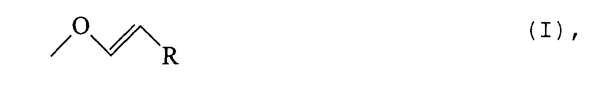 worin R Wasserstoff, Methyl oder Ethyl darstellen.

2. Ormocere gemäß Anspruch 1, **dadurch gekennzeichnet, daß** mindestens eine Siliciumverbindung
ein monomeres Silan der Formel(II) 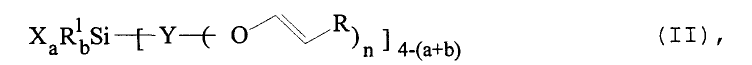 worin der Rest R unabhängig Wasserstoff, Methyl oder Ethyl, der Rest R¹ unabhängig ein aliphatischer, cycloaliphatischer oder aromatischer Rest mit 1 bis 20 Kohlenstoffatomen, der Rest X eine hydrolysierbare Gruppe und der Rest Y unabhängig einen unsubstituierte oder substituierte aliphatischen, cycloaliphatischen oder aromatischen Rest mit 1 bis 30 Kohlenstoffatome bedeuten, wobei eine oder mehrere CH₂-Gruppen durch O, C=O, -CO₂-, -SiR₂- und/ oder -SiR₂O- ersetzt sein können, und a eine ganze Zahl im Bereich von 1 bis 3, b eine ganze Zahl im Bereich von 0 bis 2 und n eine ganze Zahl im Bereich von 1 bis 3 darstellen, oder
ein cyclisches, verzweigtes oder lineares Oligo- bzw. Polysiloxan ist, umfassend Struktureinheiten der Formel (III) 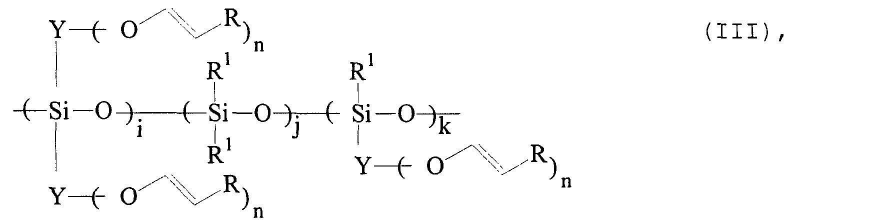 worin die Reste R, R¹ und Y sowie die Zahl n die zuvor genannte Bedeutung haben, i, j und k unabhängig eine ganze Zahl im Bereich von 0 bis 15 sind, wobei jedoch i und k nicht gleichzeitig 0 sein können.

3. Ormocer gemäß Anspruch 1 oder 2, **dadurch gekennzeichnet, daß** die hydrolysierbare Gruppe Wasserstoff, Halogen, ein Alkoxy- und/oder Acyloxy-Rest ist.

4. Ormocer gemäß einem oder mehreren der vorhergehenden Ansprüche, dadurch erhältlich, **daß** man die hydrolytische Kondensation durch einen basischen Katalysator bewirkt.

5. Ormocer gemäß Anspruch 4, **dadurch gekennzeichnet, daß** der basische Katalysator NH₃, NaOH, KOH und/oder Methylimidazol ist.

6. Ormocer gemäß einem oder mehreren der vorhergehenden Ansprüche, **dadurch gekennzeichnet, daß** man nach der Hydrolyse die Vinyletherreste der Formel (I) kationisch polymerisiert.

7. Ormocer gemäß Anspruch 6, **dadurch gekennzeichnet, daß** man die Polymerisation durch Licht initiiert.

8. Ormocer gemäß Anspruch 6 oder 7, **dadurch gekennzeichnet, daß** man vor der kationischen Polymerisation zusätzliche kationisch polymerisierbare Monomere hinzu fügt.

9. Verfahren zur Herstellung von Ormoceren gemäß einem der Ansprüche 1 bis 8, **dadurch gekennzeichnet, daß** man die Komponenten mischt, danach die Siliciumverbindungen basisch hydrolysiert und anschließend die ethylenisch ungesättigten Reste kationisch polymerisiert.

10. Verwendung von Ormoceren gemäß den Ansprüchen 1 bis 8 in Dentalwerkstoffen.
